# EUROPEAN PATENT SPECIFICATION

(11) **EP 1 146 823 B1**
(45) Date of publication and mention of the grant of the patent: **06.05.2009**
(21) Application number: 00908314.8
(22) Date of filing: 19.01.2000
(51) Int. Cl.: A61B 17/10

(54) **MODULAR LIGATING APPARATUS AND METHOD**
MODULARES LIGATIONSGERÄT UND METHODE
PROCEDE ET APPAREIL MODULAIRE DE LIGATURE

(30) Priority: 25.01.1999 US 117079 P
(43) Date of publication of application: 24.10.2001
(73) Proprietor: APPLIED MEDICAL RESOURCES CORPORATION, Rancho Santa Margarita, CA 92688 (US)
(72) Inventor: JOHNSON, Gary, M., Mission Viejo, CA 92692 (US); AHLBERG, Russell, E., Rancho Santa Margarita, CA 92688 (US)
(74) Representative: Enskat, Michael Antony Frank
(86) International application number: PCT/US2000/001296
(87) International publication number: WO 2000/042922

(56) References cited:
- EP-A- 0 612 505
- EP-A- 0 656 191
- EP-A- 0 769 275
- EP-A- 0 834 286
- US-A- 3 082 426
- US-A- 4 166 466
- US-A- 4 372 316
- US-A- 4 662 374
- US-A- 4 674 504
- US-A- 5 330 487
- US-A- 5 601 224
- US-A- 5 843 097
- US-A- 5 868 759
- US-S- D 253 611

## Description

### Background of the Invention

### Field of the Invention

This invention relates generally to ligating clip appliers and, more specially, to appliers including reusable handle assemblies and disposable multi-clip cartridges.

### Discussion of Related Art

Surgical clip appliers commonly include handles operable by a user to close jaws containing a ligating clip. The clips are mounted in the jaws in an open state, and closed around a body conduit to achieve ligation.

Clip appliers of the past have included devices that are totally reusable after steriliziation, typically in an autoclave. Such appliers, which have been particularly appreciated for their tactile feedback and "feel", have required the manual loading of the jaws with a single clip. With only a single-clip capability, at least two clip appliers have been required in the surgical environment, along with a surgical assistant who loads the appliers and shuttles them to the surgeon.

These reusable clip appliers have suffered greatly from jaw misalignment which tends to occur with repeated use. When the jaws are misaligned, the associate clips do not adequately close. In severe cases of misalignment, the clips tend to fall from the jaws before they are applied to the body conduit.

Other clip appliers, for example as disclosed in US patent number US 4,372,316, have included handle assemblies and removable cartridges containing multiple clips in a clip train. These devices have tended to be very complex as a single movement of the handle has been required to move the ultimate clip to an operable location, and to move the penultimate clip to a staging location. To accomplish these objectives, multiple hinges and linkage elements have been required which unfortunately have degraded the tactile feedback associated with the clip closure. This tactile feedback has been further degraded by a camming action which has been used to translation longitudinal movement to lateral movement associated with jaw closure.

Clearly absent from the prior art is a simplified structure, including a reusable handle assembly and disposable clip cartridge suitable for maintaining jaw alignment and tactile feedback.

### Summary of the Invention

According to the present invention there is provided a surgical clip cartridge adapted to be inserted on a pair of handles, and being operable by the handles to position a surgical clip relative to body tissue, comprising: a cartridge containing a plurality of the clips, characterised by an attachment mechanism for removably attaching the cartridge to the handles; and a pair of jaws carried by the cartridge and being adapted to receive one of the clips from the cartridge, the jaws being directly movable by operation of the handles to apply the one clip to the body tissue.The invention may also provide a clip applier incorporating the cartridge of the invention.

In accordance with the present invention, a surgical clip applier can be provided with a reusable, autoclavable handle assembly which preserves the "feel" particularly appreciated by the surgeons. A disposable clip cartridge, including a training of multiple clips, is removably attached to the handle assembly and operable by the handle assembly to individually attach the clips to a body conduit. Movement of the clip train can be limited to a pusher, a spacer, and associated linkage to provide for a simple and direct movement of the clip train. Jaws are formed as part of the disposable cartridge so that new jaws are provided with each cartridge. An interleaved structure of minimum thickness facilitates jaw alignment. The handle assembly includes arms that move laterally to produce a lateral motion in the clip jaws. The direct association between the handle assembly and the jaws provides the tactile feedback heretofore only available with single-clip appliers.

The clip applier can be adapted for use in positioning and actuating a surgical clip relative to body tissue. The applier includes a pair of handles operable by a user and the cartridge containing the plurality of the flips. The attachment mechanism removably attaches the cartridge to the handles.

### Brief Description of the Drawings

Fig. 1 is a side-elevation view of a patient with a vascular side branch and a clip applier of the present invention operable to occlude the side branch;
Fig. 2 is a perspective view of one embodiment of the clip applier;
Fig. 3 is a top-plan view of the embodiment of Figure 2;
Fig. 4 is an expanded view of the embodiment of Figure 2 showing a handle assembly and removable cartridge assembly;
Fig. 5 is a top-plan view of the cartridge assembly illustrated in Figure 4;
Fig. 6 is an expanded view of the cartridge assembly illustrated in Figure 4;
Fig. 7 is a top-plan view of the cartridge assembly illustrating direct operation of cartridge jaws by the handle assembly;
Fig. 8 is a perspective view of the interior regions of the cartridge assembly illustrating a first step in a method of operation;
Fig. 9 is a side-elevation view partially in section taken along lines 9-9 of Figure 8;
Fig. 10 is a perspective view illustrating another step in a method of operation;
Fig. 11 is a cross-section view taken along lines 11-11 of Figure 10;
Fig. 12 is a perspective view illustrating an additional step in a method of operation;
Fig. 13 is a cross-section view taken along lines 13-13 of Figure 12;
Fig. 14 is a perspective view illustrating a further step in a preferred of operation;
Fig. 15 is a cross-section view taken along lines 15-15 of Figure 14;
Fig. 16 is a perspective view of a further embodiment of the clip applier;
Fig. 17 is an expanded view of the clip applier of Figure 16 illustrating a handle assembly and removable cartridge assembly;
Fig. 18 is an expanded view of the cartridge assembly illustrated in Figure 17;
Fig. 19 is a perspective view of portions of the cartridge assembly illustrating a jaw assembly in a step associated with a method of operation;
Fig. 20 is a perspective view of interior portions of the cartridge assembly further illustrating the method step of Figure 19;
Fig. 21 is a perspective view of the interior portions of the cartridge illustrating the jaw assembly in another step of the method of operation;
Fig. 22 is a perspective view of the interior portions of the cartridge further illustrating the method step of Figure 21;
Fig. 23 is a perspective view of the interior portions of the cartridge assembly illustrating the jaw assembly in an additional step of a method of operation;
Fig. 24 is a perspective view of the interior portions of the cartridge further illustrating the method step of Figure 23;
Fig. 25 is a perspective view of the interior portions of the cartridge illustrating the jaw assembly in a further step of the method of operation;
Fig. 26 is a perspective view of the interior portions of the cartridge further illustrating the method step of Figure 25;
Fig. 27 is a perspective view of a further embodiment of the jaw assembly having floating characteristics, the view showing the jaw assembly in a fully-closed configuration;
Fig. 28 is a perspective view similar to Fig. 29 and showing the floating jaws in a fully-opened configuration; and
Fig. 29 is a perspective view of the jaw assembly illustrating an initial step of closure beginning at the distal tip of the jaws.

### Detailed Description of the Preferred Embodiments and Best Mode of the Invention

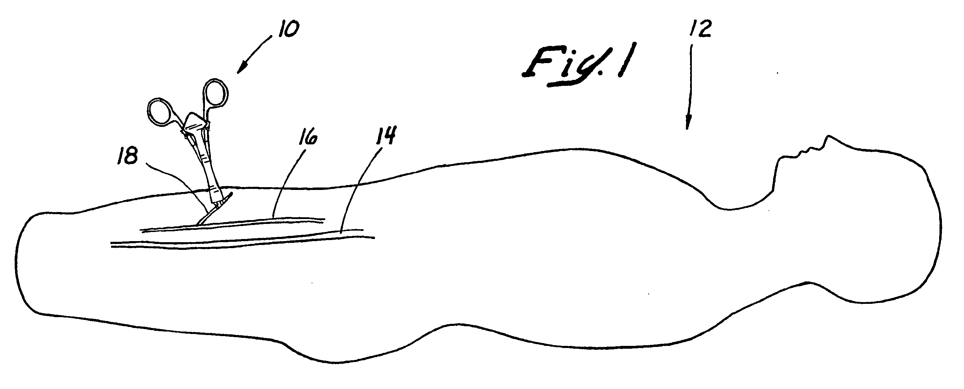

A clip applier is illustrated in Figure 1 and designated generally by the reference numeral 10. The clip applier 10 is a surgical instrument particularly adapted for use in closing or occluding blood vessels and other body conduits of a patient 12. The blood vessels may be relatively large, as in the case of the femoral artery 14, or may be relatively small, as in the case of side branches 16 of the saphenous vein 18. In each case, it is the purpose of the clip applier 10 to percutaneously engage the particular body conduit, such as the side branch 16, to position a clip along the conduit, and to crimp the clip in a closed state to occlude the conduit. The need for this type of vessel occlusion is particularly apparent in a procedure where the saphenous vein 18 is harvested to facilitate by-pass surgery. In this procedure, the side branches 16 are severed from the saphenous vein 18 and must be occluded to prevent bleeding.

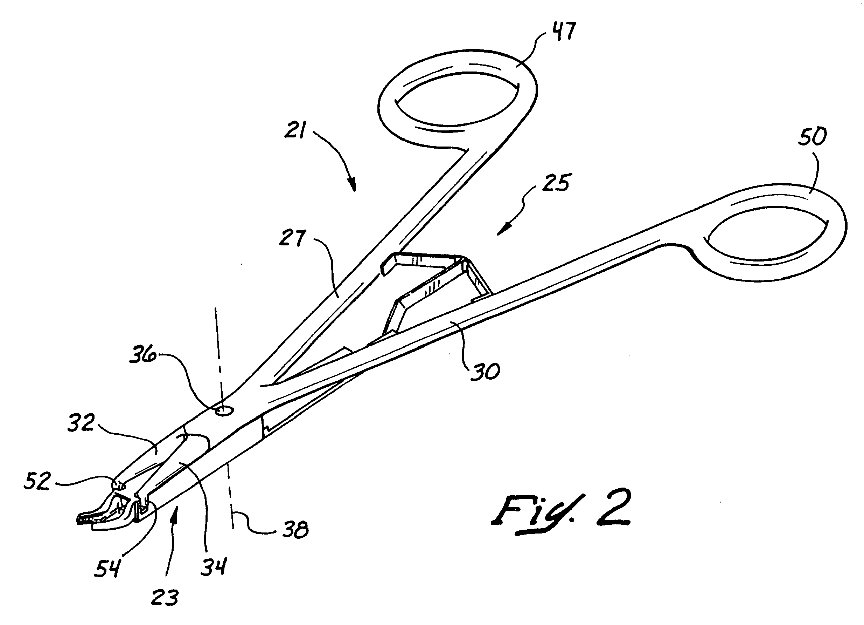

One preferred embodiment of the clip applier 10 is illustrated in the perspective view of Figure 2. In this view, it can be seen that the clip applier 10 will typically include a handle assembly 21 and a removable clip cartridge assembly 23. These two assemblies 21 and 23 are joined by linkage 25 so that operation of the handle assembly 21 also operates the cartridge assembly 23 in order to issue and crimp a clip as previously discussed. The handle assembly 21 will typically have a scissors configuration with a pair of legs 27 and 30 and a pair of arms 32 and 34 separated by a pivot point or fulcrum 36. In a common manner, the leg 27 and arm 34 form one piece of the handle assembly 21 while the leg 30 and arm 32 form a second piece of the handle assembly 21. These two pieces are joined by a fulcrum pin at the fulcrum 36, so that they pivot relative to each other about an axis 38.

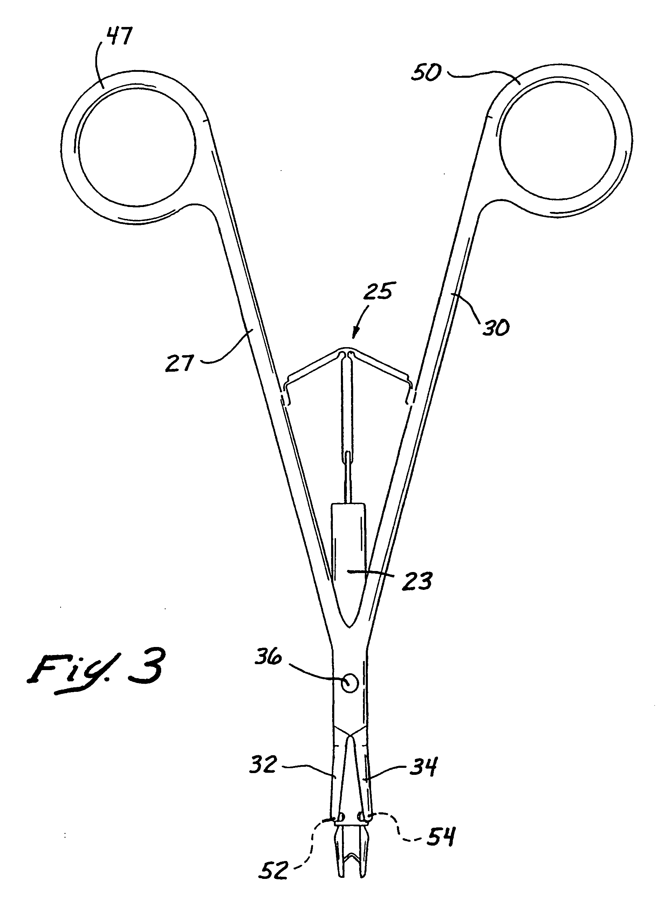

Finger-and-thumb holders 47 and 50 are provided at the proximal end of the legs 27 and 30. At the distal end of the arms 32 and 34, a pair of projections 52 and 54 extend downwardly toward the cartridge assembly 23. These projections 52 and 54 extend into the page in the top-plan view of Figure 3.

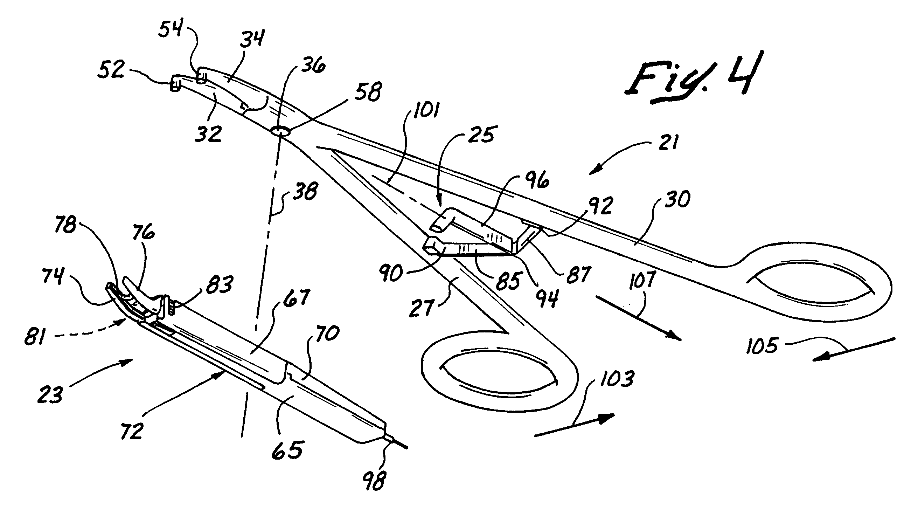

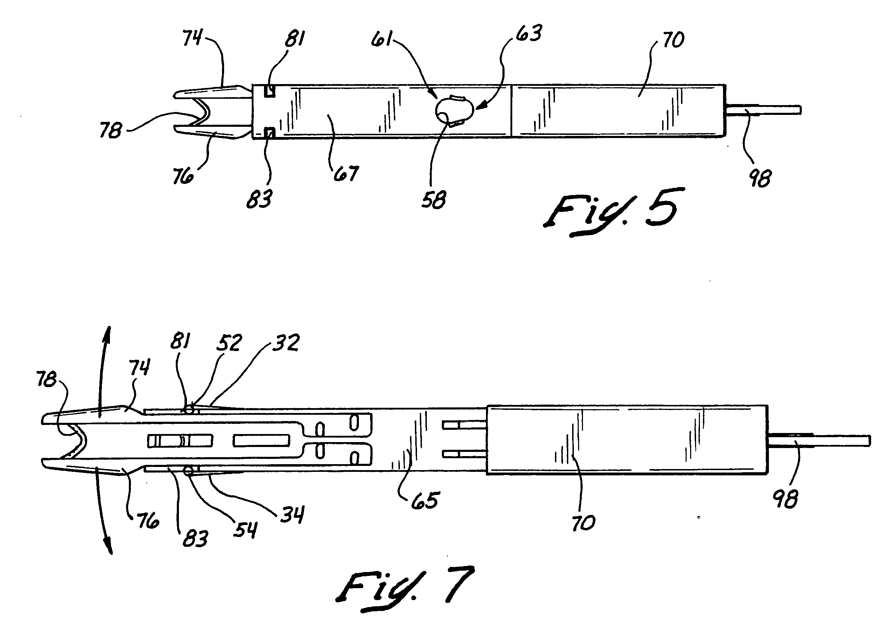

A bottom perspective view of the clip applier 10 is illustrated in Figure 4, with the handle assembly 21 separated from the cartridge assembly 23. Mounting the cartridge assembly 23 to the handle assembly 21 is facilitated by providing the bottom of the fulcrum pin 36 with an annular flange 56. This flange 56 is mateable with an opening 58 in the top of the cartridge assembly 23, as best illustrated in Figure 5. This opening 58 has an enlarged end suitable to receive the full diameter of the annular flange 56 and a smaller end for engaging and holding the annular flange 56 in the opening 58. The fulcrum pin 36 and associated annular flange 58 are suitably retained in the opening 58, to provide the cartridge assembly 23 with a removable, but generally fixed, relationship with the fulcrum pin 36. It will be noted that when the cartridge assembly 23 is fixed to the fulcrum pin 36, each of the legs 27, 30 and the arms 32, 34 is free to move relative to the cartridge assembly 23.

With further reference to Figures 4 and 5, it will be noted that the cartridge assembly 23 includes a clip tray 65 and a pair of covers 67 and 70 which form a housing 72. At the distal end of the housing 72, and a pair of jaws 74 and 76 are configured to receive a clip 78. These jaws 74 and 76 are accessible by the projections 52 and 54, respectively, of the handle assembly 21 through windows 81 and 83 in the cover 67. Importantly, operation of the handle assembly 21 causes the projections 52 and 54 to directly engage the jaws 74 and 76 without intervening parts or linkage, thereby providing a high degree of tactile feedback to the surgeon as the clip 78 is applied. In particular, it will be noted that the jaws 74 and 76 close by moving in a lateral direction. This is the same direction that the projections 52 and 54 move as they engage the jaws 74, 76 and move the jaws 74, 76 to close the clip 78. As a result, substantially all of the lateral force directed to clip closure is communicated directly to the handle assembly 21.

Figure 4 also provides a bottom-perspective view of the linkage 25, which in this embodiment includes a pair of levers 85 and 87 which engage the legs 27 and 30 through living hinges 90 and 92, respectively. The levers 85 and 87 are also joined through a living hinge 94 to a central member 96. This central member 96 is operably coupled to a pusher 98 in the cartridge assembly 23 in a manner discussed in greater detail below. With this construction, it can be seen that movement of the legs 27 and 30 of the handle assembly 21 causes the central member 96 to move the pusher 98 back and forth along an axis 101. More specifically, when the legs 27 and 30 are moved toward each other in the direction of arrows 103 and 105, the central member 96 and pusher 98 are drawn proximally along an arrow 107. Conversely, when the legs 27 and 30 are opened or separated, the member 96 and associated pusher 98 are moved distally.

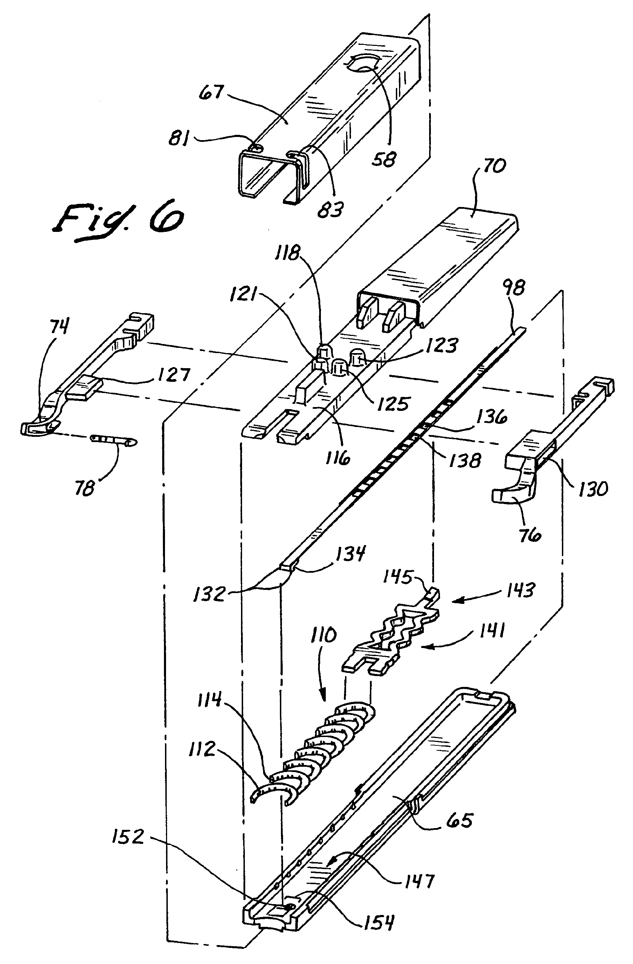

An exploded view of the cartridge assembly 23 is illustrated in Figure 6 where the tray 65, jaws 74 and 76, pusher 98, and covers 67 and 70 are separated to illustrate their detail. Also illustrated in this view is a train 110 of the clips 78 which are movable along the tray 65 and include an ultimate clip 112 and a penultimate clip 114.

From this view it can also be seen that the cover 70 is associated with a jaw support 116 having a pair of posts 118 and 121 which are adapted to receive the jaw 74, and a pair of posts 123 and 125 which are adapted to receive the jaw 76. An anti-scissoring mechanism 126 includes a flange 127 formed on the jaw 74 and an associated slot 130 provided on the jaw 76. This flange 127 mates with the slot 130 in order to maintain the jaws 74 and 76 in alignment.

The pusher 98 has an elongate configuration with the pair of flanges 132 at its distal end, which extend downwardly in Figure 6. These flanges 132 form ramps 134 which incline upwardly and proximally of the pusher 98. A plurality of openings are formed along the length of the pusher 98 and include a proximal opening 136 and an adjacent distal opening 138.

A spacer 141 is provided to move the clip train 110 along the tray 65. The length of the spacer 141 is variable between its distal end and its proximal end, and includes a projection 143 with a ramp 145 that extends upwardly and distally in Figure 6. It is the projection 143 of the spacer 141 which is adapted to be progressively received within the openings, such as the openings 136 and 138, of the pusher 98.

Of further interest in Figure 6 is a gate 147 which is provided in the distal bottom of the tray 65. This gate 147 includes a slot 152 and is pivotal on a living hinge 154 between an upper position and a lower position. In the upper position, the gate 147 extends into the path of the clip train 110. In the lower position, the gate 147 is removed from the path of the clip train 110.

The jaws 74 and 76 can be mounted on the jaw support 116 and relative to the posts 118-125 in the manner illustrated in Figure 7. This top view is of particular interest as it illustrates the relationship between the projections 52 and 54 of the handle assembly 21 and the associated jaws 74 and 76 of the cartridge assembly 23. These projections 52 and 54 extend through the windows 81 and 83, respectively, to engage the jaws 74 and 76. It is important to note that there is direct contact between the projections 52 and 54 and the associated jaws 74 and 76 without any intervening linkage, levers or camming action. This direct contact provides a significant advantage to the present invention in the form of tactile feedback which is communicated directly from the jaws to the user. This greatly facilitates the "feel" which is much appreciated by a surgeon using the clip applier 10.

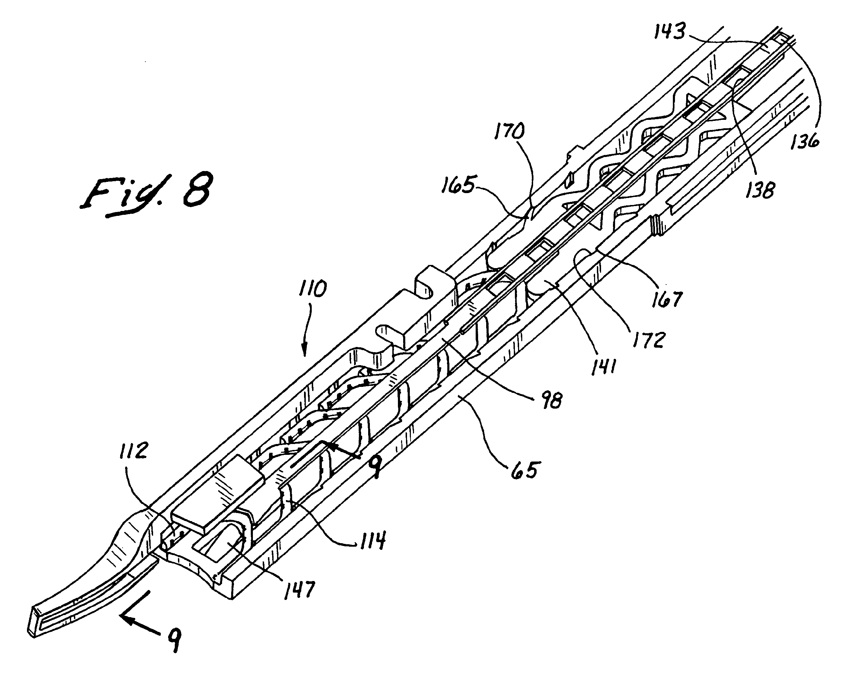

Operation of the clip applier is best discussed with reference to the progressive steps illustrated in Figures 8-12. In these views, the jaw 74 is illustrated, but its support 116 is not shown in order to expose the interior of the clip tray 65. These views are particularly beneficial in showing operation of the pusher 98 relative to the clip train 110, the gate 147, and spacer 141.

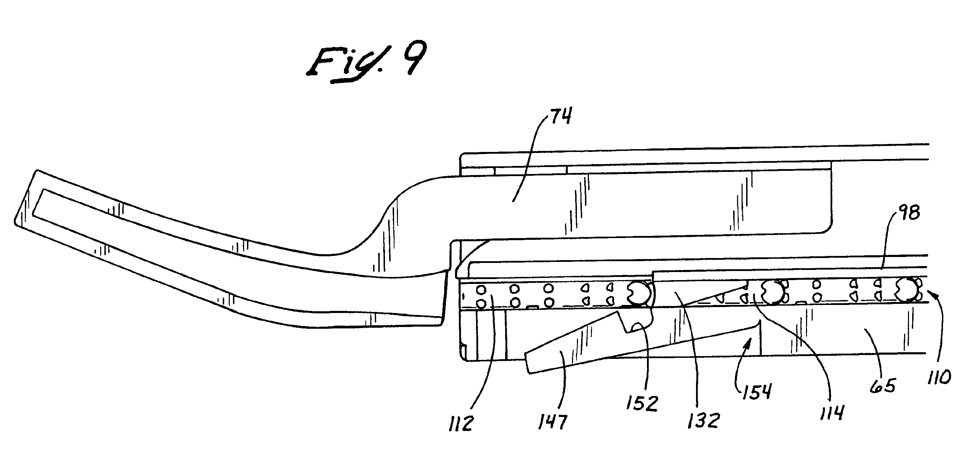

An initial position of the pusher 98 is illustrated in the perspective view of Figure 8 and the cross-section view of Figure 9. In this initial position, the distal end of the pusher 98, which includes the flanges 132, is disposed between the ultimate clip 112 and the penultimate clip 114. In this position, the flanges 132 of the pusher 98 pivot the gate 147 downwardly about its living hinge 154, as best illustrated in Figure 9. In this downward position, the gate 147 is removed from the path of the clip train 110. With the flanges 132 disposed in the path of the clip train 110, any distal movement of the clips by the spacer 141 is inhibited.

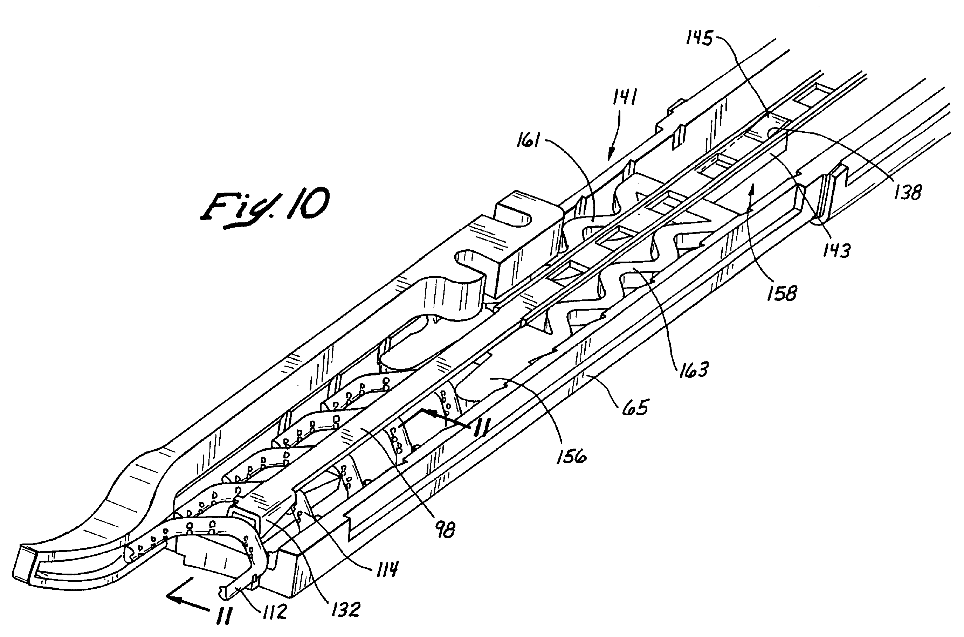

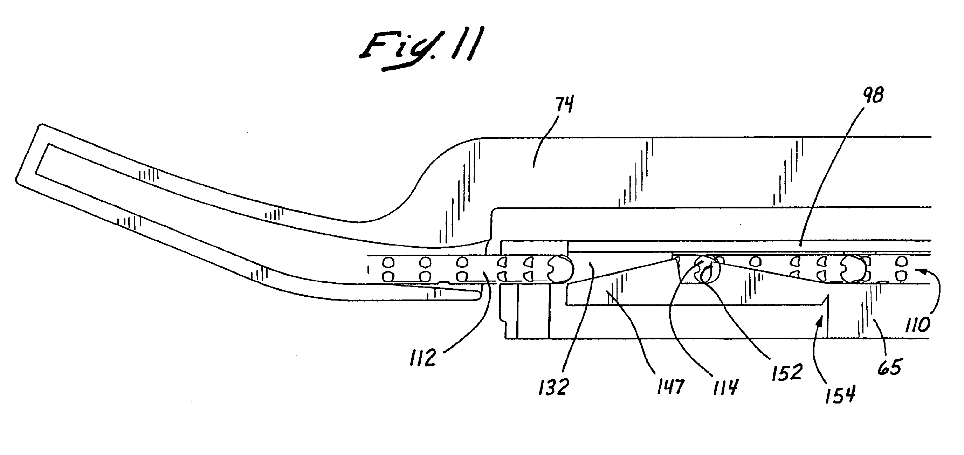

As the pusher 98 is moved distally by operation of the handle assembly 21 and linkage 25, the ultimate clip 112 is pushed by the flanges 132 distally into the jaws 74 and 76. This is best illustrated in the perspective view of Figure 10 and associated cross-sectional view of Figure 11. As the flanges 132 of the pusher 98 are moved distally, the clip train 110 is freed so that it can be moved distally by the spacer 141.

In Figure 10, the spacer 141 is illustrated to have a distal end 156 and a proximal end 158 interconnected by a pair of legs 61 and 63 which are variable in length. These legs 161 and 163 are biased to an elongate configuration, but compressible to exert a distal force on the clip train 110. The projection 143 of the spacer 141 is disposed at the proximal end 158 and adapted to be received in one of the openings, such as the opening 138, in the pusher 98. As the pusher 98 is moved distally, the proximal end 158 of the spacer 141 is also moved distally. This tends to compress the legs 161, 163, thereby exerting the distal force on the clip train 110.

It will be apparent that movement of the pusher 98 distally causes the flanges 132 to clear the gate 147 so that it is free to pivot upwardly into the path of the clip train 110. However, this upward movement of the gate 147 will be opposed by the penultimate clip 114 until it feeds in the slot 152 of the gate 147.

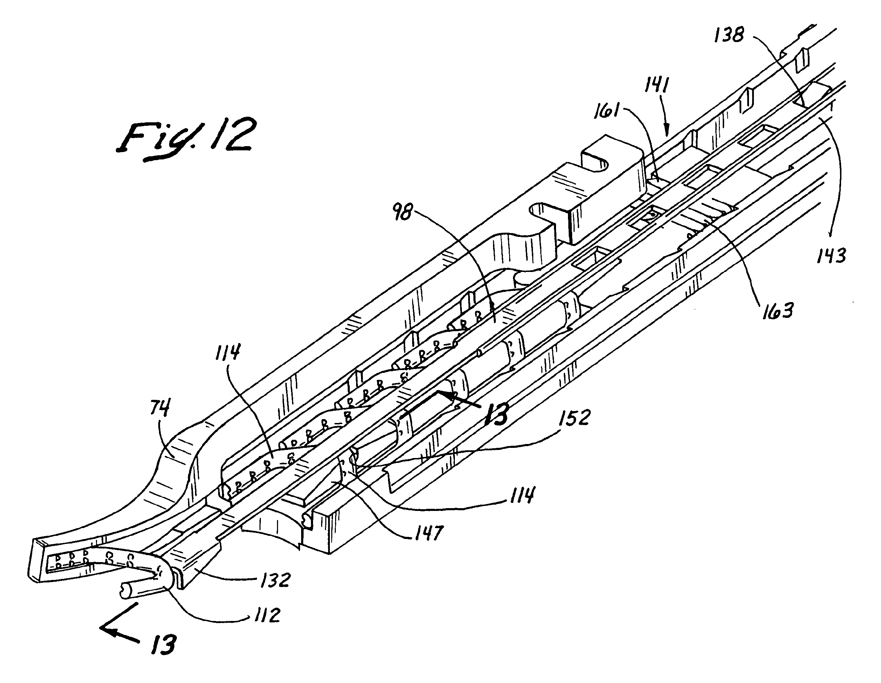

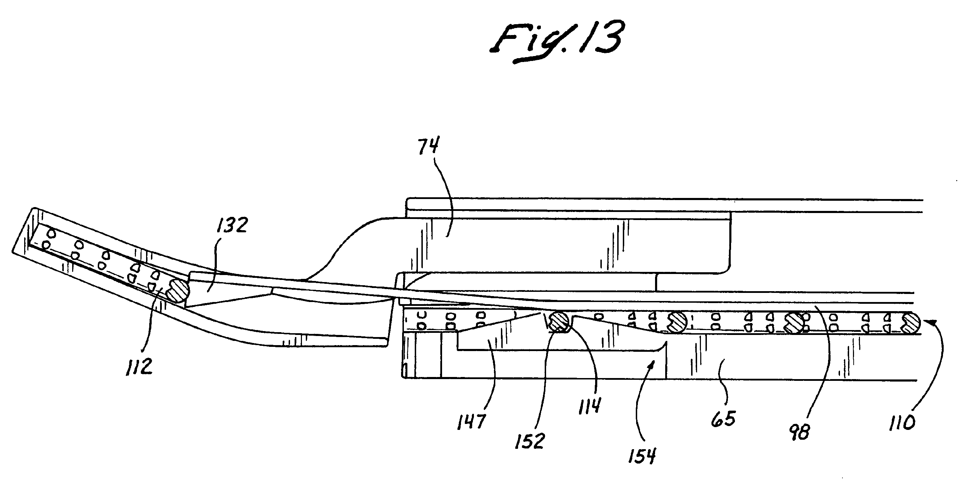

Further movement of the pusher 98 in the distal direction moves the ultimate clip 112 into its operative position at the distal end of the jaws 174 and 176. This position is best illustrated in the perspective view of Figure 12 and the associated cross-sectional view of Figure 13.

With the pusher 98 in its distal-most position, the legs 161, 163 of the spacer 141 may be fully compressed, as illustrated in Figure 12. This will create a maximum distal force on the clip train 110 operating to move the penultimate clip 114 into the slot 152, and enabling the gate 147 to rise fully into its upper-most position in the path of the clip train 110. In this position, the forward edge of the slot 152 engages the penultimate clip 114 where it is held in its gated position at the distal end of the tray 65. With the penultimate clip 114 held by the gate 147, any further movement of the clip train 110 in the distal direction is inhibited.

With the ultimate clip 112 loaded into its operative position in the jaws 74, 76, the pusher 98 can be withdrawn by operation of the handle assembly 21 and associated linkage 25. This proximal movement of the pusher 98 is caused by moving the legs 27 and 30 of the handle assembly 21 toward each other. This movement also causes the projections 52 and 54 of the associated arms 32 and 34 to directly move the jaws 74 and 76 toward each other, thereby crimping the clip 112 onto the conduit, such as the side branch 16 (Figure 1).

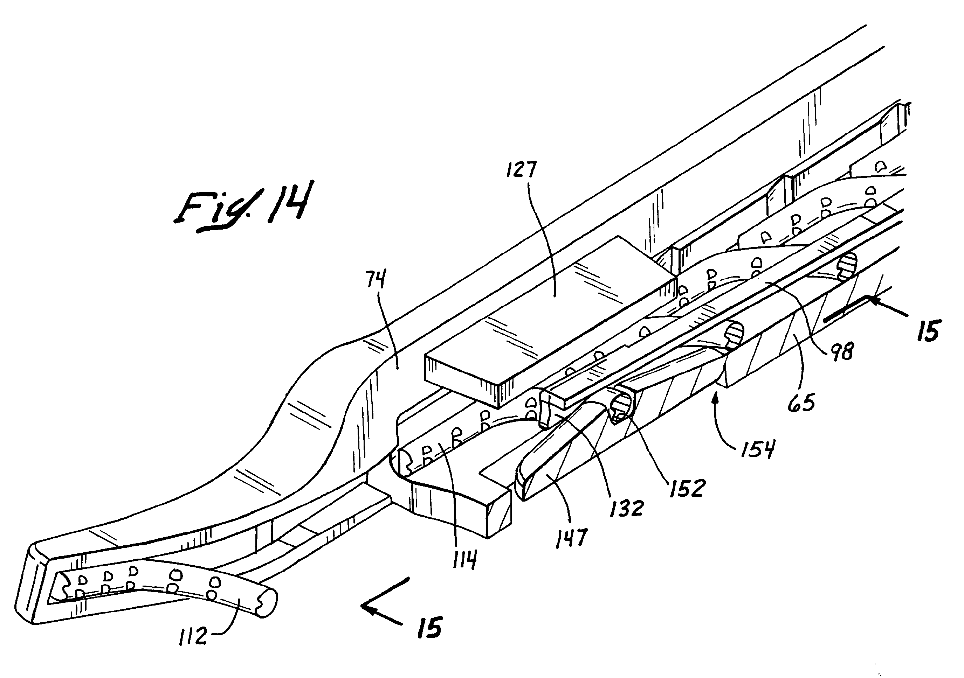

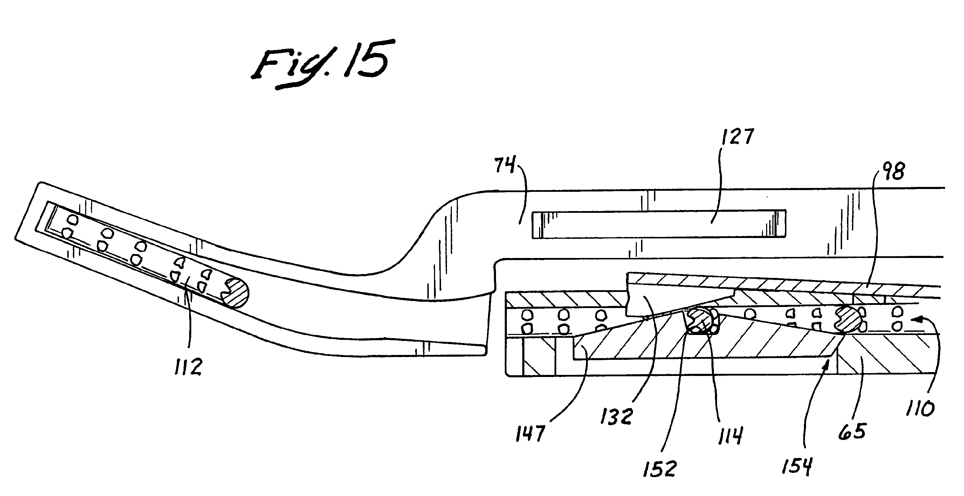

During this closure of the handle assembly 21, the pusher 98 is withdrawn in the proximal direction where it engages the clip train 110 as the ramps 134 of the flanges 132 cam over the penultimate clip 114. This is best illustrated in the perspective view of Figure 14 and associated cross-sectional view of Figure 15. This use of the clip 114 in a camming action is desirable as both the pusher 98 and the clip 114 are preferably made of metal. These materials are better suited for the high pressure sliding movement of a camming action, than a plastic material such as that preferably forming the tray 65.

Figure 14 also illustrates operation of the spacer 141 as the pusher 98 is withdrawn. In particular, it will be noted that the spacer 149 can be provided with a pair of ears 165 and 167, which progressively register with distally-facing slots 170 and 172 in the sides of the tray 65. As the distal end 156 of the spacer 141 moves distally, the ears 165, 167 progressively engage a different pair of the slots 170, 172 (best shown in Figure 8). This engagement prevents the spacer 141 from moving proximally as the pusher 98 is withdrawn. With the spacer 141 held stationary, proximal movement of the pusher 98 causes the projection 143 to withdraw from the proximal opening 136 along the ramp 145. This projection 143 slides along the pusher 98 until it snaps into the next opening, in this case the distal opening 138.

Upon further withdrawal of the pusher 98, the flanges 132 at the distal end of the pusher 98 clear the penultimate clip 114 and fall into the path of the clip train 110 again depressing the gate 147 as initially illustrated in Figures 8 and 9. From this point, the legs 27 and 30 of the handle assembly 21 can again be expanded to move the pusher 98 forward and load the penultimate clip 114 into the jaws 74 and 76.

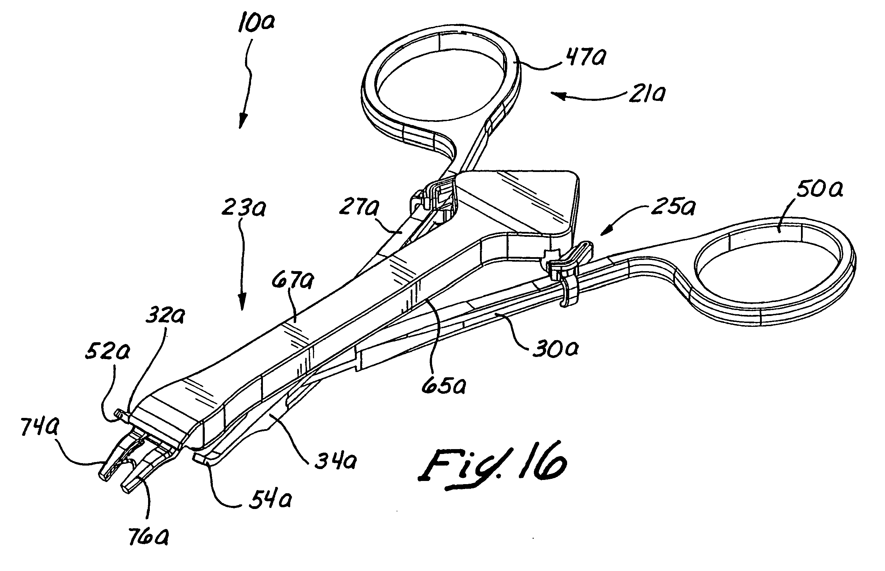

A further embodiment of the invention is illustrated in Figure 16 where elements of similar structure are designated by the same reference numerals, followed by the lower-case letter "a". By way of example, this embodiment includes a clip applier 10a with a handle assembly 21a, a cartridge assembly 23a, and associated linkage 25a. The handle assembly 21a includes finger holders 47a and 50a attached to legs 27a and 30a at the proximal end, and projections 52a and 54a attached to arms 32a and 34a, respectively, at the distal end. The cartridge assembly 23a includes a clip tray 65a and a cover 67a, which form a housing 72a. Jaws 74a and 76a extend downwardly at the distal end of the cartridge assembly 23a, in a direction opposite to that of the projections 52a and 54a.

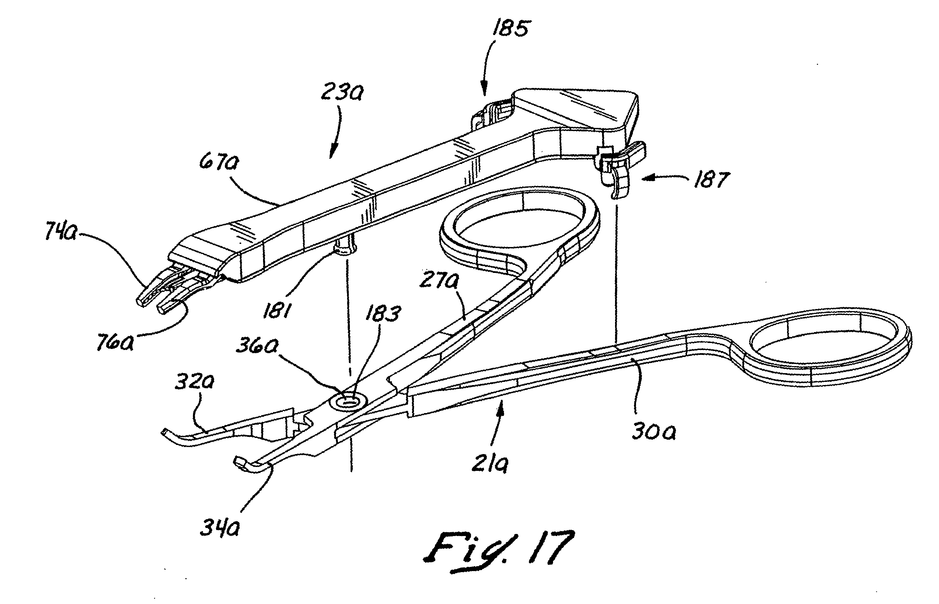

As best illustrated in Figure 17, the cartridge assembly 23a in this embodiment is attached to the handle assembly 21a by a projection 181, which can be snap-fit into a cylindrical hole 183 formed in the fulcrum 36a. Attachment of the linkage 25a to the handles 27a and 30a is facilitated by detent assemblies 185 and 187, respectively.

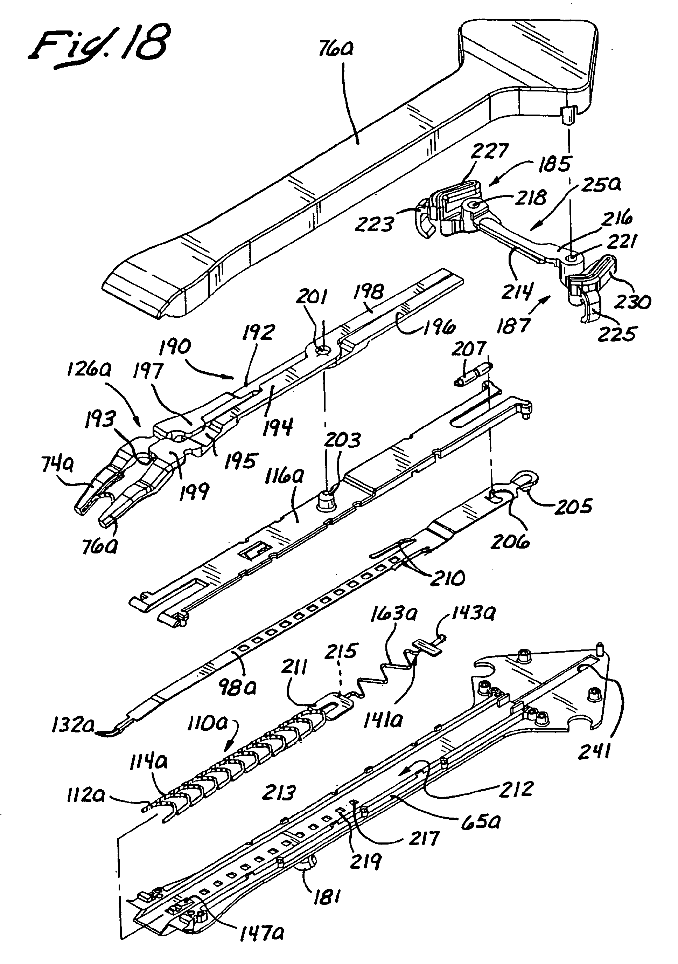

The expanded view of Figure 18 shows various other elements which are similar to those described in the embodiment of Figure 6. These elements include the jaws 74a and 76a, which in this embodiment are formed as part of a jaw assembly 190. This assembly 190 includes not only the anti-scissoring mechanism 126a, but also a pair of arms 192 and 194 which extend into an associated pair of legs 196 and 198. The arms 192, 194 are separated from the legs 196, 198 by a pivot hole 201, which is adapted to mount on a pivot post 203 associated with the jaw support 116a. In a manner disclosed in greater detail below, the arms 192, 194 have a scissoring relationship, while the legs 196 and 198 have an interfering relationship which tends to bias the jaw assembly 190 toward an open position.

Figure 18 is perhaps the best view to discuss another feature of this embodiment which is associated with the anti-scissoring mechanism 126a. This mechanism is similar to that previously discussed except that the surfaces forming the slot 130 in the Figure 6 embodiment are formed by two separate fingers 193 and 195 associated with the jaws 74a and 76a, respectively. The flange 127 of the Figure 6 embodiment is also separated into a pair of fingers 197 and 199 associated with the jaws 74a and 76a, respectively. These finger pairs 193, 195 and 197, 199 are interleaved in order to prevent any scissoring of the arms 192 and 194 and associated jaws 74a and 76a. Thus, the finger 193 is disposed beneath the finger 199, thereby preventing any upward movement of the jaw 74a relative to the jaw 76a. Similarly, the finger 197 is disposed over the finger 195 to prevent any downward movement of the jaw 74a relative to the jaw 76a.

The pusher 98a is similar to that previously discussed with the flanges 132a at the distal end. A pin 205 extends downwardly from the pusher 98a in the same direction as the flanges 132a, but at the proximal end of the pusher 98a. A tab 206 is also provided at the proximal end of the pusher 98 to receive one end of a tension spring 207, the other end of which is fixed to the cover 76a. The spring 207 tends to bias the pusher 198 in the distal direction. The pusher 98a can also be provided with a pair of leaf springs 210, which are biased outwardly to engage ratchets and cams on the lateral surfaces of the cover 76a. This structure insures completion of a full clip cycle before initiating a subsequent cycle. This is accomplished by moving the leaf springs 210 along a ratchet in the proximal direction and then along a cam in the proximal direction.

This embodiment also includes the clip train 110a with an ultimate clip 112a and penultimate clip 114a. This clip train 110a is moved in a distal direction by the spacer 141a, which in this embodiment has but a single leg 163a of variable length. As in the previous embodiment, the projection 143a is provided at the proximal end of the spacer 141a. The clip train 10a and associated spacer 141a move along a clip train path 212 in the tray 65a. The gate 147a can be provided at the distal end of this path 212 in the manner previously described.

The spacer 141a in this embodiment includes a distal U-shape member 211, but differs in several respects from the spacer 141 previously described. For example, the ears 165 and 167 of the spacer 98 which register with the slots 170 and 172 of the tray 65, respectively, are replaced by a single tooth 215 on the bottom side of the member 211, which register progressively with slots, such as the slots 217 and 219, in the tray 65a. The tooth 215 and associated slots, such as the slots 217 and 219, function as a ratchet mechanism 220 to permit distal movement of the member 211 while inhibiting rearward movement of the member 211.

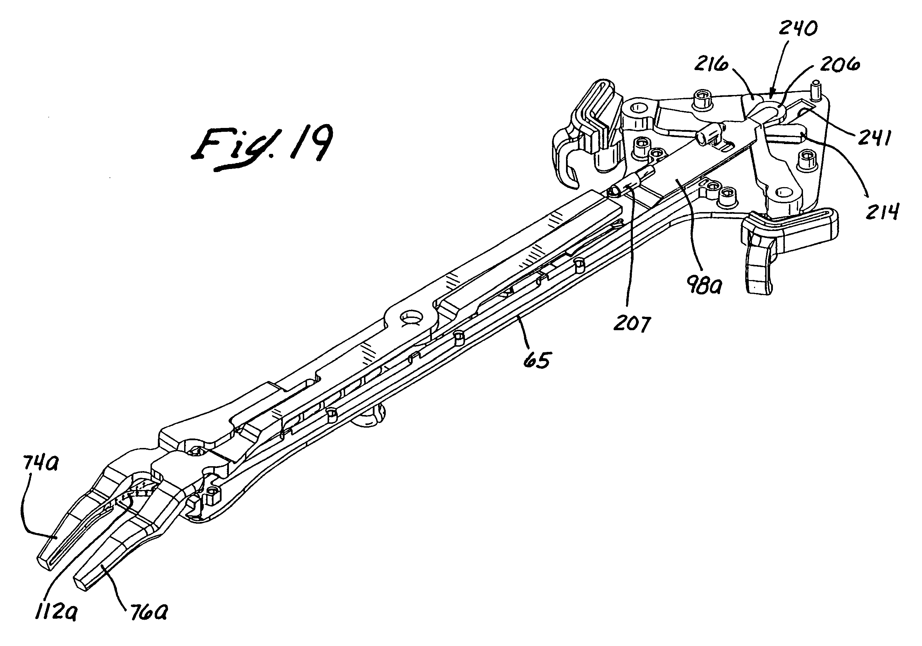

The linkage 25a of this embodiment include the detent assemblies 185 and 187, which respectively engage the handles 27a and 30a, as previously disclosed. These assemblies 185 and 187 are each attached to an associated lever arm 214 and 216, thus the detent assembly 185 pivots with the arm 214 about a fulcrum 218. Similarly, the detent assembly 187 pivots with the arm 216 about a fulcrum 221. Each of the detent assemblies 185 and 187 includes a cupped flange 123 and 125, and a support 127 and 130, respectively, which engage the associated leg 27a and 30a of the handle assembly 21a. The flange supports 227 and 230 are relatively long to facilitate mounting the flanges 223 and 225, but are bent back on themselves to provide a short moment arm relative to the associated fulcrums 218 and 221, respectively. Operation of the resulting linkage 25a produces a progressive scissoring action of the lever arms 214 and 216 as the associated legs 27a and 30a of the handle assembly 21a are moved relative to each other. This operation of the linkage 25a will be better understood with a description of the progressive steps of operation and reference to the associated drawings of Figures 19-26.

Figure 19 is a perspective view of the cartridge assembly 23a with the cover 67a removed to expose the jaw assembly 190. This view illustrates a moment in time when the ultimate clip 112a is about to be loaded into the jaws 74a and 76a. The handle assembly 21 a has been closed bringing the legs 27a and 30a into close proximity, along with the attached detent assemblies 185 and 187 associated with the linkage 25. This close proximity has caused the arms 214 and 216 to scissor forming a variable V-groove 240 which moves along a slot 241 at the proximal end of the tray 65a. The pin 240 at the proximal end of the pusher 98a seats in this V-groove and slot 241, and is carried proximally with the pusher 98a, as illustrated in Figure 19. This movement of the pusher 98a relative to the cover 76a expands the spring 207 thereby biasing the pusher 98a in a distal direction. Upon release of the leaf springs 210 from the associated ratchet, in the manner previously discussed, the pusher 98a is free to move under the bias of the spring 207 pushing the ultimate clip 212a into the slots of the jaws 74a and 76a.

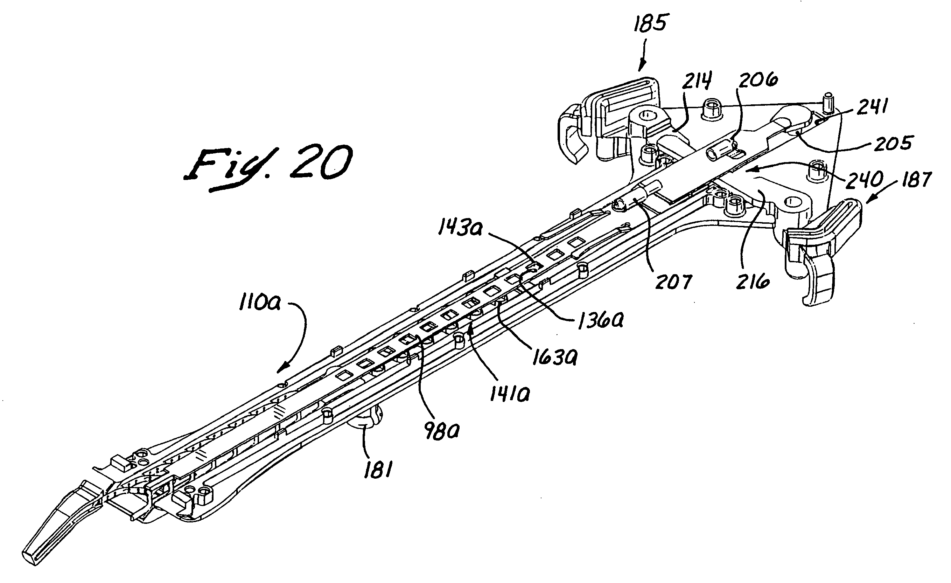

The same step of operation is illustrated in Figure 19 and in Figure 20 where the jaw assembly 190 has been removed to expose the pusher 98a and clip train 110a. Note that the presence of the projection 143a of the spacer 141a in the distal hole 136 of the pusher 98a begins compression of the spacer 141a which biases the clip train 110a in the distal direction.

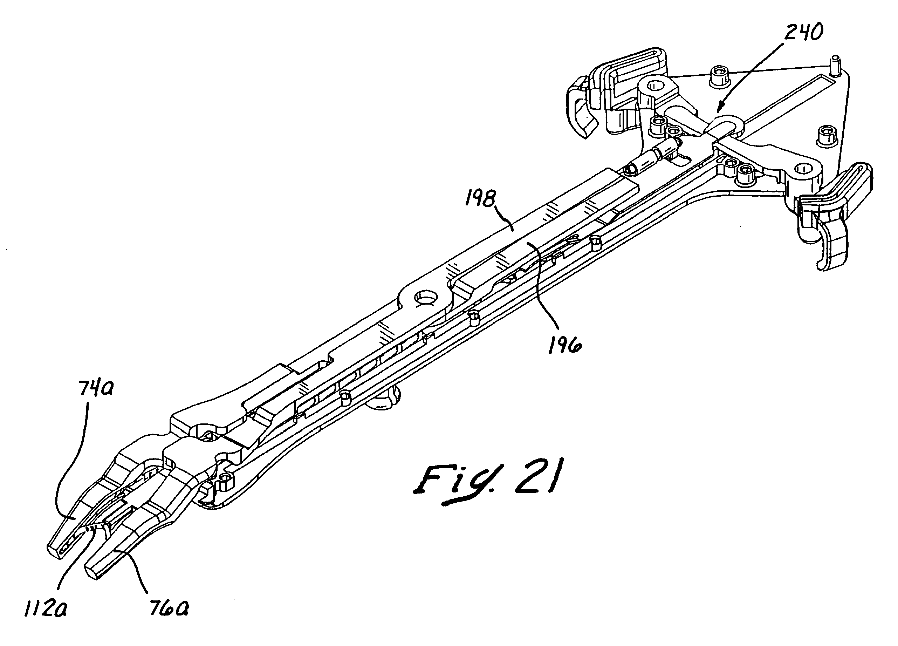

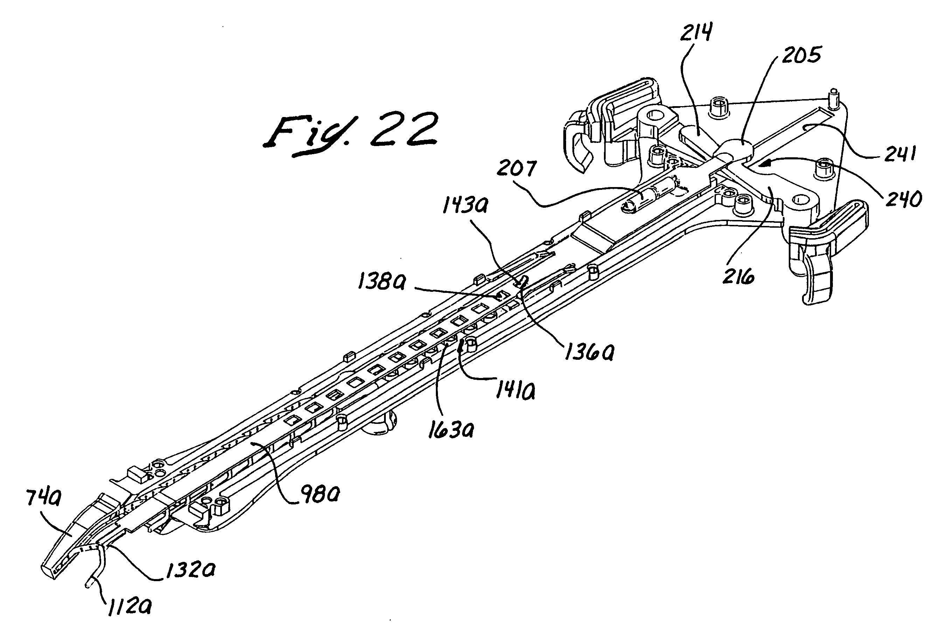

Figures 21 and 22 illustrate a step of operation where the ultimate clip 112a is moved to its operative position within the jaws 74a and 76a. Figure 21 illustrates the jaw assembly 190 with the jaws 74a and 76a fully separated to receive the clip 112a. In this state, the interfering relationship between the legs 196 and 198 is relieved with the jaws 74a and 76a maximally separated.

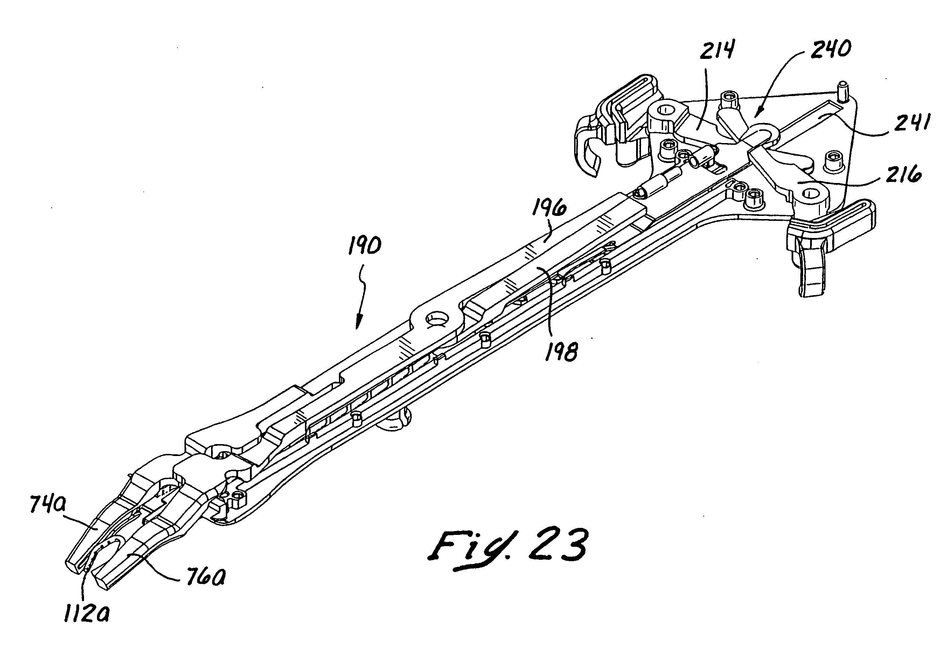

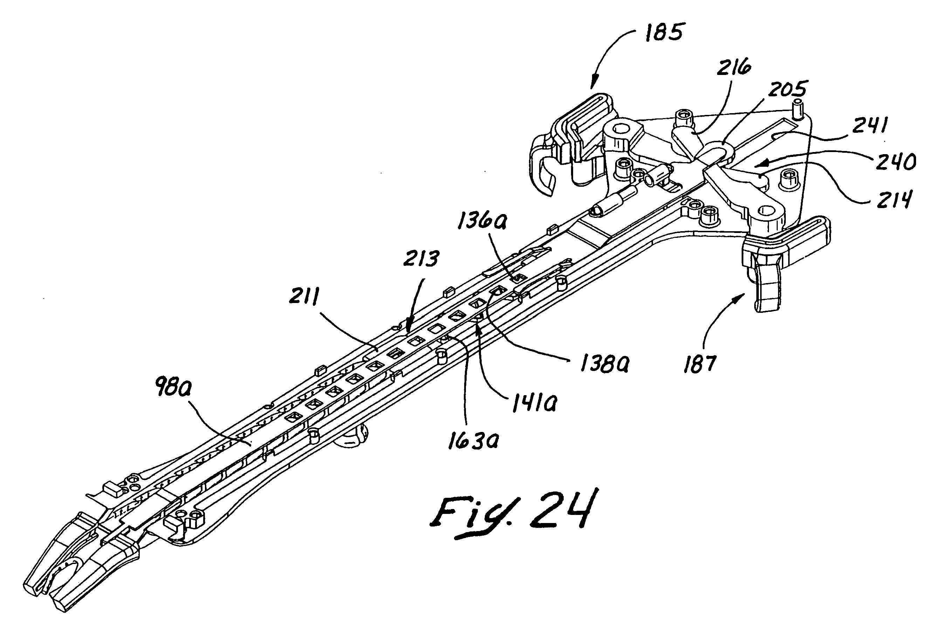

The next step in the method of operation is illustrated in Figures 23 and 24. After the ultimate clip 112a has been loaded in its operative position in the jaws 74a and 76a, the handle assembly 21a can be moved to a closed position bringing the legs 27a and 30a into closer proximity. In the manner previously discussed, this causes the arms 32a and 34a of the handle assembly 21a to also move into proximity, directly pressing the projections 52a and 54a (Figure 16) against the respective jaws 74a and 76a. In this manner, operation of the handle assembly 21a begins to close the jaws 74a, 76a, clipping the clip 112a. With reference to Figure 23, it will be noted that the legs 196 and 198 associated with jaw assembly 190 are forced against each other as the jaws 74a and 76a are closed. The interfering fit, which prevents any scissoring action of the legs 196 and 198, causes these legs to bend slightly, thereby biasing the jaws 74a and 76a to the open position. Further closure of these jaws increases the bending and, therefore, the bias associated with the interfering legs 196 and 198.

Referring now to Figure 24, it will be noted that closure of the handle assembly 21a also moves the detent assemblies 185 and 187 into closer proximity. This causes the assemblies 185 and 187 to pivot slightly on the associated fulcrums 218 and 221, thereby causing the associated lever arms 214 and 216 to move the V-groove proximally along the slot 241. The pin 205 is carried in this V-groove proximally along the slot 241 drawing with it the remainder of the pusher 98a. This withdraws the flanges 132a over the penultimate clip 114a, spreads the spring 207, and begins movement of the leaf springs 210 along the associated ratchet.

In the previous step, illustrated in Figure 22, the projection 143a associated with the spacer 141 a was seated in the proximal hole 136a of the pusher 98a. As the pusher 98a is drawn proximally, in the step illustrated in Figure 23, it tends to draw the projection 143a and hence the spacer 141 a in the proximal direction. However, this tendency has different effects at the different ends of the spacer 141a. At the proximal end, the projection 143a will move with the hole 136a a short distance as the leg 163a expands to its normal state. At the distal end of the spacer 141a, the U-shape member 211 cannot move proximally due to the action of the ratchet 213. With the U-shape member 211 held stationary, and the projection 143a moving proximally, the leg 163a expands, and the spacer 141a elongates. This occurs until the leg 163a is pulled beyond its natural state, at which point the projection 143a is withdrawn from the hole 136a. Further proximal movement of the spacer 98a causes this projection 143a to snap into the distal hole 138a.

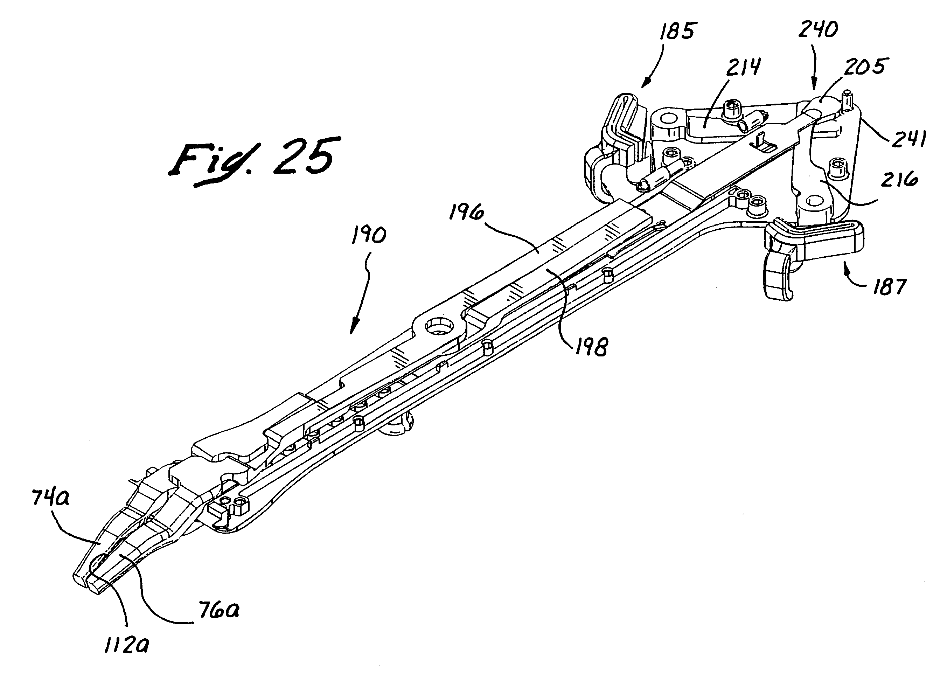

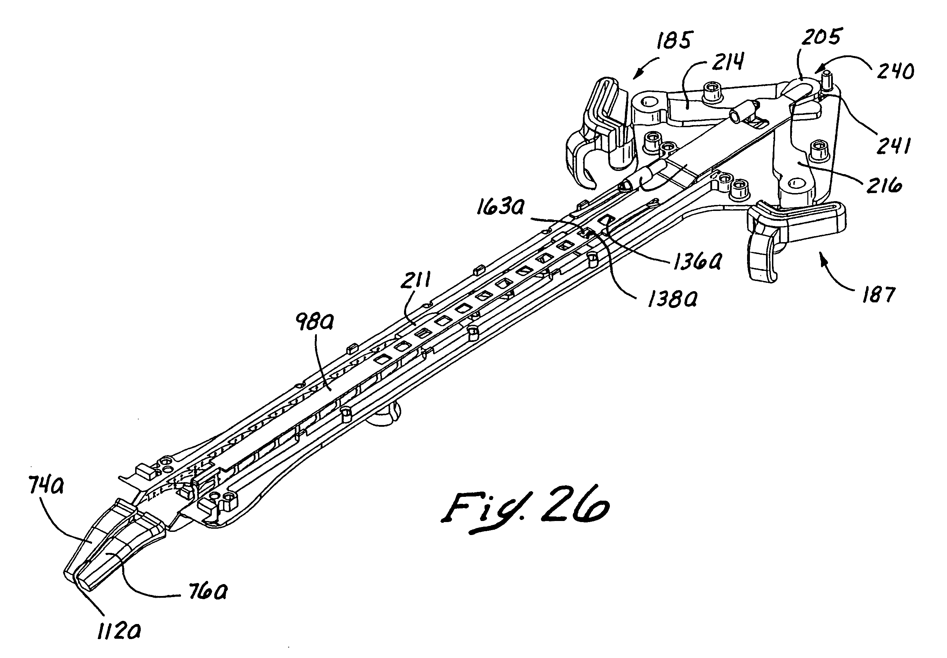

The next step in the operative process is illustrated in Figure 25 and 26 where the ultimate clip 212 is fully compressed. This occurs primarily by operation of the handle assembly 121a where the legs 27a and 30a (Figure 16) are fully closed. By direct contact between the arms 32a and 34a (Figure 16) and the jaw assembly 190, the jaws 74a and 76a are also fully closed to crimp the clip 112a. With the jaws 74a and 76a fully closed, the legs 196 and 198 associated with the jaws assembly 190 are fully strained to maximize the bias tending the open the jaws 74a and 76a.

With reference to Figure 26, it can be seen that the full closure of the handle assembly 121a (Figure 16) operates to move the detent mechanisms 185 and 187 into their most proximal relationship. This causes the lever arms 214 and 216 to rotate proximally so that the V-groove 240 carries the projection 205 toward the proximal end of the slot 241. This draws the pusher 98a to its most proximal position moving the spring 207 to its fully expanded state.

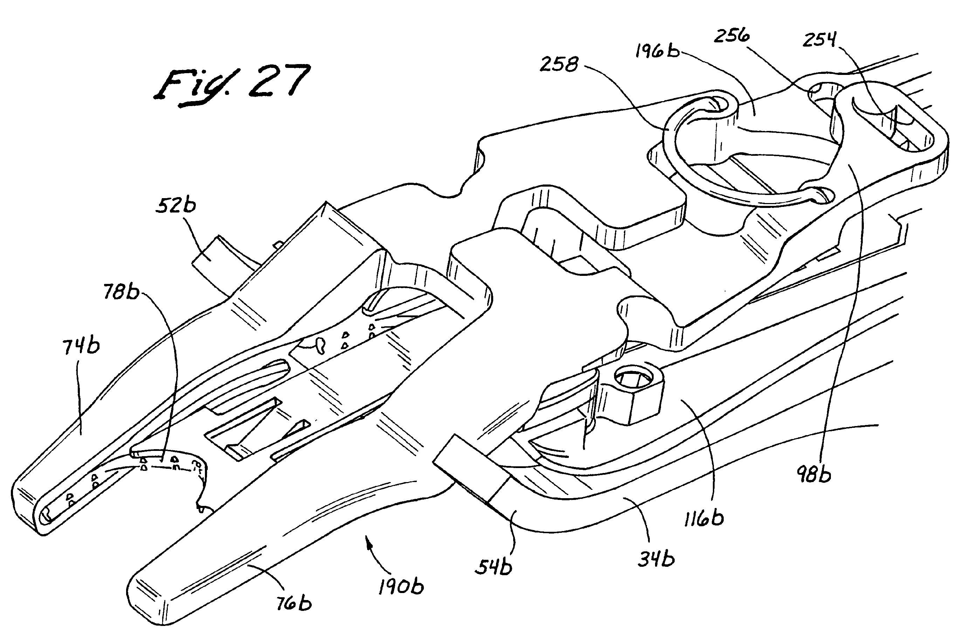

Another embodiment of the jaw assembly is illustrated in Figures 27-29 where elements of structure similar to those previously discussed are designated by the same reference numerals, followed by the lower-case letter "b". For example, in Figure 27, the jaw assembly 190b includes the jaws 74b and 76b and associated legs 196b and 198b, with the anti-scissoring mechanism 126b disposed there between. As in the previous embodiment, this jaw assembly 190b can be mounted on the jaw support 116b, but in this case, only a single post 250 is required. The arms 32b and 34b and associated projections 52b and 54b, of the handle assembly 21b, are also illustrated in Figure 27.

This jaw assembly 190b differs from those previously discussed, as it includes a floating hinge structure 252 disposed at the proximal end of the assembly 190b. This floating hinge structure 252, in the illustrated embodiment, includes a pair of slots 254 and 256 which extend laterally at the proximal end of the respective legs 196b and 198b. The single post 250 extends into each of the slots 254 and 256, but has a width which is less than the lateral extension of the slots 254 and 256. As a result, the proximal end of the legs 196b and 198b are free to move laterally or float from a first position wherein the post 250 engages the inner ends of the slots 254, 256, to a second position wherein the post 250 engages the outer ends of the slots 254, 256. A leaf spring 258 can be provided to bias the legs 196b and 198b to the first position where they are maximally separated and the post 250 engages the inner ends of the slots 254, 256 as illustrated in Figure 27.

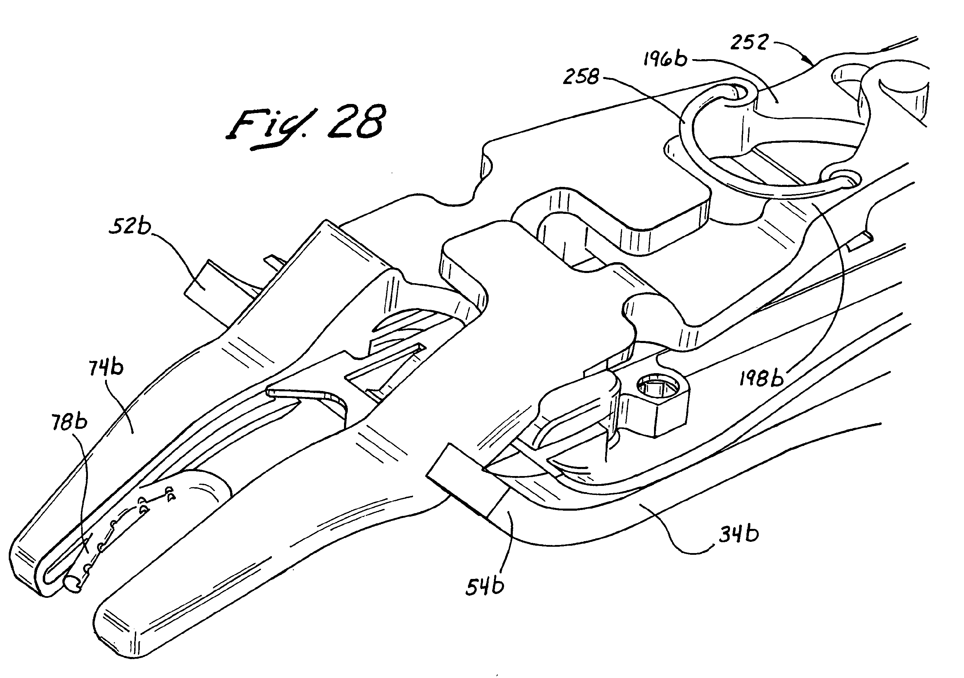

This structure of the jaw assembly 190b is of particular advantage as it permits closure of the jaws 174b, 176b in a method which insures that the associated clip 78b is closed first at its distal end. This closure process begins when the projections 52b and 54b of the arms 32b and 34b are moved laterally against the jaws 74b and 76b, respectively. This step in the closure process is best illustrated in Figure 28 where the distal tip of the jaws 74b and 76b are moved together, while the spring 258 maintains the floating hinge 252 in its first expanded position. With the legs 196b and 198b maintained in an expanded, generally-spaced relationship, and the tips of the jaws 74b and 76b brought into proximity, the distal end of the clip 78b is generally closed, while the proximal end of the clip 78b is maintained generally open.

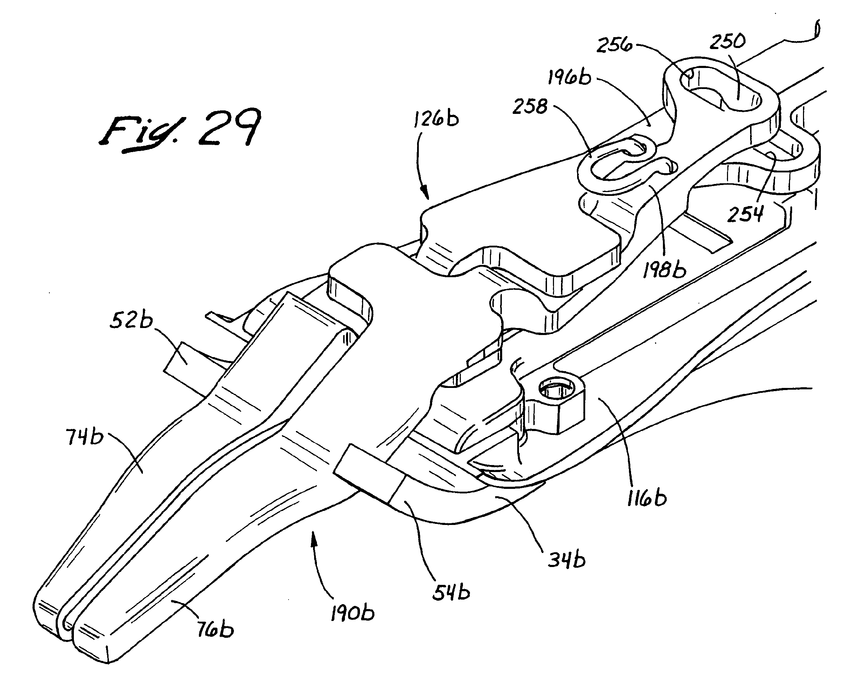

With the tips of the jaws 74b and 76b closed, they provide a pivot point which causes the further pressure of the projections 52b and 54b on the jaws to close the proximal end of the legs 196b and 198b. This closure is accommodated by the floating hinge 252, which moves to its second, contracted configuration, as best illustrated in Figure 29. With the distal end of the jaws 74b and 76b closed, this closure of the proximal ends causes the proximal end of the clip 78b to be closed, thereby forming the clip 78b into its fully-closed configuration. This preferred method of operation is facilitated in a preferred embodiment wherein the post 250 has a diameter of .060 inches, and the slots 254 and 256 each have an axial width of .060 inches and a lateral length of .140 inches. It will be apparent that this embodiment, including the single post 250 and two slots 254 and 256, could be replaced by multiple posts each associated with one of the legs 196b and 198b and movable within individual slots or the same slot in the jaw support 116b.

Having discussed the preferred embodiments of the concept and their methods of operation, it is apparent that the clip applier 10a provides significant advantages over previous structures. The surgeons will particularly appreciate the "feel" associated with the device. This "feel" is enhanced by the non-disposable scissor assembly 21 which provides a weight and structure familiar to the surgeon. The disposable clip assembly 23 is easily mounted on this structure to provide for multiple clip applications. The "feel" is further enhanced by the direct pressure of the scissor assembly 21 on the jaws 74 and 76. The mechanism of the cartridge assembly 23 is of ultimate simplicity and comprised primarily of only two operative members, namely the pusher 98 and the spacer 141. Of these two elements, only the pusher 98 is actively involved as the spacer 141 is provided with two ratcheting components which cause it to move forwardly by operation of the pusher 98. This forward movement is enhanced by the expandable leg 163 so that the spacer 141 tends to function passively and with a motion similar to that of an inchworm.

The active motion of the pusher 98 is facilitated by the distal bias of the compression spring 207. Proximal movement of the pusher 98 is accomplished by the movement of the V-groove 240, defined by the arms 214 and 216, along the slot 241.

Although the foregoing embodiments and methods of operation have been described in significant detail, it will be apparent that this invention is a concept which may be otherwise embodied. As a result, one is cautioned not to determine the nature of the concept solely with reference to the described embodiments and method steps, but rather with particular reference to the following claims.

## Claims

1. A surgical clip cartridge adapted to be inserted on a pair of handles, and being operable by the handles to position a surgical clip (78) relative to body tissue, comprising:
a cartridge (23) containing a plurality of the clips (78); and
an attachment mechanism (56, 58) for removably attaching the cartridge (23) to the handles; **characterised by**
a pair of jaws (74, 76) carried by the cartridge (23) and being adapted to receive one of the clips (78) from the cartridge, the jaws (74, 76) being directly movable by operation of the handles to apply the one clip (78) to the body tissue.

2. A cartridge according to Claim 1, **characterised in that** the jaws (74, 76) are attached to the cartridge and form with the cartridge a cartridge assembly (23) removably mountable on the handles.

3. A cartridge according to Claim 1, **characterised in that** the jaws (74, 76) are movable by operation of the handles between a first jaw position, to receive the one clip (78), and a second jaw position, to apply the one clip (78) to the body tissue.

4. A cartridge according to Claim I, **characterised by**:
a jaw assembly including a pair of arms disposed relative to the handles and each extending distally to an associated one of the pair of jaws.

5. A cartridge according to Claim 4, **characterised in that** the arms of the jaw assembly include metal, and the jaws of the jaw assembly include plastics.

6. A cartridge according to Claim 1, **characterised by**:
a linkage (96, 98) carried by the cartridge and adapted to engage the handles; and
the linkage (96, 98) being responsive to operation of the handles to advance the one clip (78) from the cartridge into the jaws (74, 76).

7. A clip applier for applying a clip to an object, the clip applier comprising a clip cartridge as claimed in any one of Claims 1 to 5,
a handle assembly (21) having a pair of opposing legs (27, 30) and a pair of opposing arms (32, 34), the legs (27, 30) being operable to move the arums (32, 34) pivotally about a fulcrum (36);
**characterised in that** the arms (32, 34) of the handle assembly are positioned to directly engage the jaws (74, 76) without intervening linkage, to close the jaws (74, 76) by operation of the legs (27, 30) of the handle assembly, to apply the one clip (78) to the object.

8. A clip applier according to Claim 7, **characterised in that** the clip cartridge (23) is mounted to the handle assembly at the fulcrum (36).

9. A clip applier according to Claim 7, **characterised in that** the clip cartridge (23) is mounted to the handle assembly (21) in a pivotal relationship with each of the legs (27, 30) and the arms (32, 34) of the handle assembly.

10. A clip applier according to Claim 9, **characterised in that** the clip cartridge (23) is mounted to the handle assembly (21) and has a length which extends generally between the opposing legs (27, 30) of the handle assembly and the opposing arms (32, 34) of the handle assembly.

11. A clip applier according to Claim 7, **characterised in that**
the handle assembly (21) includes a fulcrum pin disposed at the fulcrum; and
the cartridge is connected to the fulcrum pin of the handle assembly.

12. A clip applier according to Claim 7, **characterised in that**
the jaws (74, 76) move in a lateral direction to close the clip (78); and
the arms (32, 34) of the jaw assembly move substantially in the lateral direction to engage the jaws (32, 34) and to close the jaws on the clip.

13. A clip applier as claimed in Claim 7, further comprising:
a linkage (25) carried by one of the handles and the cartridge assembly, the linkage being responsive to lateral movement of the handles to produce longitudinal movement of the clips (78) within the cartridge.

14. A clip applier according to Claim 13, **characterised in that** the linkage (25a) is formed as part of the cartridge assembly and is removably coupled to the handles.

15. A clip applier according to Claim 13, **characterised in that** the linkage (25) is formed as part of the handles and removably coupled to the cartridge assembly.

16. A clip applier according to Claim 13, **characterised in that** the cartridge assembly further comprises a clip pusher (98) longitudinally movable within the cartridge to individually issue the clips (78), the clip pusher (98) being coupled by the linkage (25) to the handles.

17. A clip applier according to Claim 13, wherein the linkage (25a) is attached to the handle and the clip pusher (98) is removably coupled to the linkage.

18. A clip applier according to Claim 13, **characterised in that:**
the jaws include a first jaw and a second jaw opposing the first jaw; and
the first jaw (76) includes portions defining a slot (130) and the second jaw (74) includes a tab (127) movable within the slot to maintain the first jaw in alignment with the second jaw.

19. A clip applier according to Claim 13, **characterised in that:**
the handles are movable generally in a plane having a first side and a second side;
the jaws (74,76) are curved to extend distally in the direction of the first side of the plane; and
the cartridge is removably attached to the handles on the first side of the plane.

20. A clip applier according to Claim 13, wherein the linkage (25a) includes at least one mounting bracket (85,87) for releasably engaging an associated one of the handles, the bracket being variable in size to accommodate handles of different diameter.

21. A clip applier according to Claim 20, wherein the mounting bracket includes a living hinge.

## Patentansprüche

1. Chirurgischer Klammereinsatz, der angepasst ist, auf einem Griffpaar eingeschoben zu werden und durch die Griffe bedienbar ist, um eine chirurgische Klammer (78) relativ zum Körpergewebe zu positionieren, umfassend:
Einen Einsatz (23), der eine Pluralität der Klammern (78) enthält, und
einen Befestigungsmechanismus (56, 58) zum entfernbaren Befestigen des Einsatzes (23) an die Griffe; **gekennzeichnet durch**
ein Paar vom Einsatz (23) getragener Einspannbacken (74, 76) und die angepasst sind, eine der Klammern (78) vom Einsatz zu empfangen, wobei die Einspannbacken (74, 76) **durch** Betrieb der Griffe direkt bewegbar sind, um die eine Klammer (78) auf das Körpergewebe anzuwenden.

2. Einsatz nach Anspruch 1, **dadurch gekennzeichnet, dass** die Einspannbacken (74, 76) am Einsatz befestigt sind und mit dem Einsatz eine Einsatzbaugruppe (23) bilden, die entfernbar an den Griffen montierbar ist.

3. Einsatz nach Anspruch 1, **dadurch gekennzeichnet, dass** die Einspannbacken (74, 76) durch Betrieb der Griffe zwischen einer ersten Einspannbackenposition, um die eine Klammer (78) zu empfangen und einer zweiten Einspannbackenposition, um die eine Klammer (78) auf das Körpergewebe anzuwenden, bewegbar sind.

4. Einsatz nach Anspruch 1, **gekennzeichnet durch**:
Eine Einspannbackenbaugruppe, die ein Paar Arme umfasst, die relativ zu den Griffen angeordnet sind und sich distal jeweils zu einer zugehörigen Einspannbacke des Einspannbackenpaars erstrecken.

5. Einsatz nach Anspruch 4, **dadurch gekennzeichnet, dass** die Arme der Einspannbackenbaugruppe Metall einschließen und die Einspannbacken der Einspannbackenbaugruppe Kunststoffe einschließen.

6. Einsatz nach Anspruch 1, **gekennzeichnet durch**:
Eine Verbindung (96, 98), die vom Einsatz getragen wird und angepasst ist, in die Griffe einzugreifen; und
wobei die Verbindung (96, 98) auf Betrieb der Griffe anspricht, um die eine Klammer (78) aus dem Einsatz in die Einspannbacken (74, 76) vorzuschieben.

7. Klammeranwendungsgerät zur Anwendung einer Klammer auf ein Objekt, wobei das Klammeranwendungsgerät einen Klammereinsatz, wie in einem der Ansprüche 1 bis 5 beansprucht,
eine Griffbaugruppe (21) mit einem Paar gegenüber liegender Schenkel (27, 30) und ein Paar gegenüberliegender Arme (32, 34) umfasst, wobei die Schenkel (27, 30) betriebsfähig sind, die Arme (32, 34) um einen Drehpunkt (36) schwenkbar zu bewegen;
**dadurch gekennzeichnet, dass** die Arme (32, 34) der Griffbaugruppe positioniert sind, ohne dazwischenkommende Verbindung, direkt in die Einspannbacken (74, 76) einzugreifen, um die Einspannbacken (74, 76) durch Betätigung der Schenkel (27, 30) der Griffbaugruppe zu schließen, um die eine Klammer (78) auf das Objekt anzuwenden.

8. Klammeranwendungsgerät nach Anspruch 7, **dadurch gekennzeichnet, dass** der Klammereinsatz (23) am Drehpunkt (36) an die Griffbaugruppe montiert ist.

9. Klammeranwendungsgerät nach Anspruch 7, **dadurch gekennzeichnet, dass** der Klammereinsatz (23) in einer drehgelenkigen Beziehung zu jedem der Schenkel (27, 30) und den Armen (32, 34) der Griffbaugruppe an die Griffbaugruppe (21) montiert ist.

10. Klammeranwendungsgerät nach Anspruch 9, **dadurch gekennzeichnet, dass** der Klammereinsatz (23) an die Griffbaugruppe (21) montiert ist und eine Länge hat, die sich generell zwischen den gegenüberliegenden Schenkeln (27, 30) der Griffbaugruppe und den gegenüberliegenden Armen (32, 34) der Griffbaugruppe erstreckt.

11. Klammeranwendungsgerät nach Anspruch 7, **dadurch gekennzeichnet, dass:**
die Griffbaugruppe (21) einen am Drehpunkt angeordneten Drehbolzen umfasst; und
der Einsatz mit dem Drehbolzen der Griffbaugruppe verbunden ist.

12. Klammeranwendungsgerät nach Anspruch 7, **dadurch gekennzeichnet, dass:**
sich die Einspannbacken (74, 76) in Querrichtung bewegen, um die Klammer (78) zu schließen; und
sich die Arme (32, 34) der Einspannbackenbaugruppe im Wesentlichen in der Querrichtung bewegen, um die Einspannbacken (32, 34) in Eingriff zu bringen und die Einspannbacken an der Klammer zu schließen.

13. Klammeranwendungsgerät nach Anspruch 7, das weiter umfasst:
Eine von einem der Griffe getragene Verbindung (25) und die Einsatzbaugruppe, wobei die Verbindung auf Querbewegung der Griffe anspricht, um Längsbewegung der Klammern (78) innerhalb des Einsatzes zu erzeugen.

14. Klammeranwendungsgerät nach Anspruch 13, **dadurch gekennzeichnet, dass** die Verbindung (25a) als Teil der Einsatzbaugruppe gebildet und entfernbar an die Griffe gekoppelt ist.

15. Klammeranwendungsgerät nach Anspruch 13, **dadurch gekennzeichnet, dass** die Verbindung (25) als Teil der Griffe gebildet und entfernbar an die Einsatzbaugruppe gekoppelt ist.

16. Klammeranwendungsgerät nach Anspruch 13, **dadurch gekennzeichnet, dass** die Einsatzbaugruppe weiter einen Klammerdrücker (98) umfasst, der innerhalb des Einsatzes in Längsrichtung bewegbar ist, um die Klammern (78) einzeln auszugeben, wobei der Klammerdrücker (98) durch die Verbindung (25) an die Griffe gekoppelt ist.

17. Klammeranwendungsgerät nach Anspruch 13, wobei die Verbindung (25a) am Griff befestigt ist und der Klammerdrücker (98) entfernbar an die Verbindung gekoppelt ist.

18. Klammeranwendungsgerät nach Anspruch 13, **dadurch gekennzeichnet, dass:**
Die Einspannbacken eine erste Einspannbacke und eine zweite Einspannbacke umfassen, die der ersten Einspannbacke gegenüberliegt; und
die erste Einspannbacke (76) Teile einschließt, die einen Schlitz (130) definieren und die zweite Einspannbacke (74) eine Nase (127) umfasst, die innerhalb des Schlitzes bewegbar ist, um die Ausrichtung der ersten Einspannbacke mit der zweiten Einspannbacke beizubehalten.

19. Klammeranwendungsgerät nach Anspruch 13, **dadurch gekennzeichnet, dass:**
die Griffe generell in einer Ebene bewegbar sind, die eine erste Seite und eine zweite Seite aufweist;
die Einspannbacken (74, 76) gekrümmt sind, um sich distal in die Richtung der ersten Seite der Ebene zu erstrecken; und
der Einsatz entfernbar an den Griffen auf der ersten Seite der Ebene befestigt ist.

20. Klammeranwendungsgerät nach Anspruch 13, wobei die Verbindung (25a) zumindest einen Montageträger (85, 87) zum lösbaren in Eingriff bringen eines zugehörigen Griffs der Griffe umfasst, wobei der Träger in der Größe variabel ist, um Griffe verschiedenen Durchmessers aufzunehmen.

21. Klammeranwendungsgerät nach Anspruch 20, wobei der Montageträger ein 'Living-Hinge' umfasst.

## Revendications

1. Une cartouche d'agrafes chirurgicales adaptée pour être insérée sur une paire de manches et étant opérable par les manches de manière à positionner une agrafe chirurgicale (78) relativement au tissu corporel, comprenant :
une cartouche (23) contenant une pluralité d'agrafes chirurgicales (78) et
un mécanisme d'attache (56, 58) pour attacher la cartouche de façon amovible (23) aux manches ; **caractérisée par**
une paire de mâchoires (74, 76) portée par la cartouche (23) et étant adaptée pour recevoir l'une des agrafes (78) de la cartouche, les mâchoires (74, 76) étant directement mobiles par l'opération des manches de manière à appliquer la une agrafe chirurgicale (78) au tissu corporel.

2. Cartouche selon la Revendication 1, **caractérisée en ce que** les mâchoires (74, 76) sont attachées à la cartouche et forment avec la cartouche un ensemble de cartouche (23) montable de façon amovible sur les manches.

3. Cartouche selon la Revendication 1, **caractérisée en ce que** les mâchoires (74, 76) sont mobiles par l'opération des manches entre une première position de mâchoire, de manière à recevoir l'agrafe (78) et une deuxième position de mâchoire, de manière à appliquer l'agrafe (78) au tissu corporel.

4. Cartouche selon la revendication 1, **caractérisée par**:
un ensemble de mâchoire comprenant une paire de bras disposée relativement aux manches et chacun s'étendant distalement jusqu'à une mâchoire associée de la paire de mâchoires.

5. Cartouche selon la Revendication 4, **caractérisée en ce que** les bras de l'ensemble de mâchoire renferment du métal et les mâchoires de l'ensemble de mâchoire renferment du plastique.

6. Cartouche selon la Revendication 1, **caractérisée par** :
une liaison (96, 98) portée par la cartouche et adaptée pour engager les manches ; et
la liaison (96, 98) étant responsive à l'opération des manches pour faire avancer l'agrafe (78) de la cartouche dans les mâchoires (74, 76).

7. Applicateur d'agrafes pour l'application d'une agrafe à un objet, l'applicateur d'agrafes comprenant une cartouche d'agrafes selon l'une quelconque des Revendications 1 à 5.
un ensemble de manche (21) possédant une paire de jambes opposée (27, 30) et une paire de bras opposée (32, 34), les jambes (27, 30) étant opérables pour déplacer les bras (32, 34) en pivotement sur un point d'appui (36) ;
**caractérisé en ce que** les bras (32, 34) de l'ensemble de manche sont positionnés de manière à engager directement les mâchoires (74, 76) sans s'interposer dans la liaison, de sorte à fermer les mâchoires (74, 76) par l'opération des jambes (27, 30) de l'ensemble de manche, afin d'appliquer l'agrafe (78) à l'objet.

8. Applicateur d'agrafes selon la Revendication 7, **caractérisé en ce que** la cartouche d'agrafes (23) est montée sur l'ensemble de manche au niveau du point d'appui (36).

9. Applicateur d'agrafes selon la Revendication 7, **caractérisé en ce que** la cartouche d'agrafes (23) est montée sur l'ensemble de manche (21) en relation pivotable avec chacune des jambes (27, 30) et les bras (32, 34) de l'ensemble de manche.

10. Applicateur d'agrafes selon la Revendication 9, **caractérisé en ce que** la cartouche d'agrafes (23) est montée sur l'ensemble de manche (21) et possède une longueur qui s'étend généralement entre les jambes opposées (27, 30) de l'ensemble de manche et les bras opposés (32, 34) de l'ensemble de manche.

11. Applicateur d'agrafes selon la Revendication 7, **caractérisé en ce que**
l'ensemble de manche (21) comprend un pivot disposé au niveau du point d'appui et la cartouche est connectée au pivot de l'ensemble de manche.

12. Applicateur d'agrafes selon la Revendication 7, **caractérisé en ce que**
les mâchoires (74, 76) se déplacent dans une direction latérale de manière à fermer l'agrafe (78) ; et
les bras (32, 34) de l'ensemble de mâchoire se déplacent sensiblement dans la direction latérale de manière à engager les mâchoires (32, 34) et fermer les mâchoires sur l'agrafe.

13. Applicateur d'agrafes selon la Revendication 7, comprenant en outre :
une liaison (25) portée par l'un des manches et l'ensemble d'agrafes, la liaison étant responsive au mouvement latéral des manches de manière à produire un mouvement longitudinal des agrafes (78) au sein de la cartouche.

14. Applicateur d'agrafes selon la Revendication 13, **caractérisé en ce que** la liaison (25a) est formée en tant que faisant partie intégrante de l'ensemble de cartouche et est couplée de façon amovible aux manches.

15. Applicateur d'agrafes selon la Revendication 13 **caractérisé en ce que** la liaison (25) est formée en tant que faisant partie intégrante des manches et est couplée de façon amovible à l'ensemble de cartouche.

16. Applicateur d'agrafes selon la Revendication 13 **caractérisé en ce que** l'ensemble de cartouche comprend en outre un pousseur d'agrafes (98) longitudinalement mobile au sein de la cartouche de sorte à éjecter individuellement les agrafes (78), le pousseur d'agrafes (98) étant couplé par la liaison (25) aux manches.

17. Applicateur d'agrafes selon la Revendication 13, la liaison (25a) étant attachée au manche et le pousseur d'agrafes (98) étant couplé de façon amovible à la liaison.

18. Applicateur d'agrafes selon la Revendication 13, **caractérisé en ce que :**
les mâchoires comprennent une première mâchoire et une deuxième mâchoire opposée à la première mâchoire ; et
la première mâchoire (76) comprend des parties définissant une fente (130) et la deuxième mâchoire (74) comprend une patte (127) mobile au sein de la fente afin de maintenir la première mâchoire en une relation d'alignement avec la deuxième mâchoire.

19. Applicateur d'agrafes selon la Revendication 13, **caractérisé en ce que :**
les manches sont généralement mobiles dans un plan ayant un premier côté et un deuxième côté ;
les mâchoires (74, 76) sont incurvées de manière à se prolonger distalement dans la direction du premier côté du plan ; et
la cartouche est attachée de façon amovible aux manches sur le premier côté du plan.

20. Applicateur d'agrafes selon la Revendication 13, la liaison (25a) comprenant au moins un support de montage (85, 87) pour l'engagement libérable d'un manche associé des manches, le support étant de taille variable de manière à recevoir des manches de diamètres différents.

21. Applicateur d'agrafes selon la Revendication 20, le support de montage comprenant une jointure ferme.
